# EUROPEAN PATENT SPECIFICATION

(11) **EP 3 773 895 B1**
(45) Date of publication and mention of the grant of the patent: **20.04.2022**
(21) Application number: 19717371.9
(22) Date of filing: 27.03.2019
(51) Int. Cl.: A61N 5/10, A61B 8/00, A61B 8/08

(54) **RADIOTHERAPY SYSTEM**
STRAHLENTHERAPIESYSTEM
SYSTÈME DE RADIOTHÉRAPIE

(30) Priority: 04.04.2018 IT 201800004199; 27.04.2018 IT 201800004953; 10.08.2018 IT 201800008048
(43) Date of publication of application: 17.02.2021
(73) Proprietor: S.I.T. - Sordina IORT Technologies S.p.A., 36100 Vicenza (VI) (IT)
(72) Inventor: FELICI, Giuseppe, 36100 VICENZA (IT); DI FRANCESCO, Massimo, 36100 VICENZA (IT)
(74) Representative: Burchielli, Riccardo
(86) International application number: PCT/IT2019/050067
(87) International publication number: WO 2019/193616

(56) References cited:
- EP-A1- 0 647 457
- EP-A1- 2 818 116
- EP-A2- 2 233 099
- WO-A1-01/06924
- US-A1- 2004 122 311
- US-A1- 2007 081 709
- US-A1- 2008 033 283
- US-A1- 2008 177 279
- US-A1- 2011 009 742
- US-A1- 2012 016 316
- US-A1- 2014 187 955
- US-A1- 2016 121 142
- US-A1- 2017 209 716
- US-A1- 2018 053 335

## Description

### Technical field

This invention relates to a system for radiotherapy treatments.

The system is particularly suitable for Intraoperative Radio Therapy treatment (IORT or IOeRT) of oncological patents.

### Background Art

Intraoperative Radio Therapy (IORT) consists in subjecting the tumour bed or the tumour residue to radiation during the surgical procedures.

This technique allows the dose to the healthy tissue to be minimised and the dose to the target to be maximised, thanks to the possibility to insert special screens in the surgical incision, that is to say, the possibility to mobilise and move the healthy tissue and/or the organs at risk.

IORT has become established over recent years thanks to the development of mobile accelerators, designed to carry out the treatment directly in the operating room; it is currently performed substantially as follows.

The target to be treated is identified visually by the surgical-radiotherapy team, without any real-time diagnostics by specific images.

The docking, that is, the positioning of the radiation applicator on the target, is performed manually, without the certainty of correct positioning on the target nor the assistance of a dedicated robotic system.

The authors of this invention consider that this positioning is very imprecise: in fact, it is necessary to consider that the deposition of the dose of radiation on the target and on the adjacent tissue is considerably influenced by the positioning, and in particular by the orientation of the applicator in space, the applicator usually being several tens of centimetres in length.

The dose of radiation, on the target and on the organs which are healthy or in any case to be protected, is estimated through the following assumptions:
- the tissue subjected to radiation as well as the adjacent healthy tissue and/or the organs at risk are homogenous and isotropic; and
- the tissue is water-equivalent.

The authors of this invention consider that the current calculation of the dose of radiation is also relatively imprecise, due also to the fact that the very large number of diagnostic possibilities with techniques such as magnetic resonance and computed tomography cannot currently be used during a surgical procedure: in fact, it is not possible to introduce a patient with an open surgical incision in a magnetic resonance or TAC apparatus.

The authors have noted that these imprecisions currently complicate execution of the IORT, very often limiting the use substantially just to breast tumours, despite its undoubted clinical effectiveness for the treatment of tumours in general, as also confirmed by the most recent ASTRO and NCCN guidelines.

The authors of this invention also consider that the current procedures and equipment for performing IORT limit the planning of any post-surgical treatment where the IORT is executed as a boost.

Similarly, the authors consider that the current technologies do not allow full use of the potentials of investigation techniques such as magnetic resonance, axial computed tomography, positron emission tomography or single photon emission tomography, for chemical or metallurgic processes on mechanical parts, components or electrical or electronic devices of relatively large size, in particular when it would be desirable to perform operations close to the scanner - which also has relatively large dimensions - for magnetic resonance, tomography or which in any case has performed the above-mentioned investigations inside the part.

In particular, prior art documents US2004/122311 A1 and US2014/187955A1 disclose systems for performing radiotherapy treatments having the technical features of the preamble of the appended claim 1.

An aim of the invention is to overcome the above-mentioned drawbacks and in particular to determine and apply the dose of radiation of a radiological treatment with a greater precision compared with known processes, using more effectively, with respect to the currently known techniques, the advantages and the precision of techniques for investigating the internal structure of bodies such as magnetic resonance, computed axial tomography, radiological stratigraphy, positron emission or single-photon emission tomography, ultrasound, Doppler ultrasound, radiography, fluoroscopy, angiography, scintigraphy.

### Disclosure of the Invention

This aim is achieved with a radiotherapy system according to the appended claims, which can be used in a process for performing radiotherapies,

According to a particular example, of the process, the position detection subsystem (11) determines the position in space of the plurality of images (IM_1, IM_2 ... IM_i... IM_N) - preferably ultrasound images - and/or, if necessary, the above-mentioned virtual model of the inside of the body of the patient (P) according to the same reference system of linear and/or angular coordinates (X, Y, Z; α, β, γ; X', Y', Z'; α', β', γ'), according to which the movement system (7) and/or the position detection subsystem (11) determines the position and/or movements in space of the radiation head (3).

According to a particular example, the process comprises the operation of converting, by means of a suitable logic unit, the position in space of the plurality of images (IM_1, IM_2 ... IM_i... IM_N) - preferably ultrasound images - from the reference system (X, Y, Z, α, β, γ) originally used by the position detection subsystem (11) to the reference system (X', Y', Z', α', β', γ') originally used by the movement system (7).

According to a particular example, the process comprises the operation of converting, by means of a suitable logic unit, the position in space of the radiation head (3) from the reference system (X', Y', Z', α', β', γ') used originally by the movement system (7) to the reference system (X, Y, Z, α, β, γ) used originally by the position detection subsystem (11).

According to a particular example of the process, a virtual model comprises one or more two-dimensional images or a three-dimensional model of the inside of the body to be treated (P), such as, for example, a numerical virtual model.

According to a particular example of the process, on the basis of the virtual model (IM_1, IM_2, IM_i, IM_N) and by means of the movement system (7), the radiation head (3) is moved to perform a radiotherapy treatment or other predetermined radiological treatment on said body to be treated (P).

According to a particular example of the process, the body to be treated (P) is immobilised on the treatment support (15) whilst it is scanned or otherwise examined by the diagnostics subsystem for images (22).

According to the invention, the aim is achieved with a radiotherapy system having the characteristics according to claim 1.

According to a particular embodiment of the system (1), the radiation head (3) is designed for emitting one or more of the following radiations: photons, X-rays, gamma rays, alpha rays, protons, ions, ionizing rays.

According to a particular embodiment of the system (1, 1', 1"), the distance detection system (120) comprises one or more of the following systems for measurement of distances and dimensions: an optical system, for example stereoscopic, a radar system with electromagnetic and/or acoustic waves, a LASER radar system.

According to a third aspect of the invention, this aim is achieved with a computer program having the characteristics according to claim 6.

A fourth and a fifth aspect of the present disclosure relate to obtaining a virtual three-dimensional model of the inside the body of a patient (P) starting from a plurality of substantially two-dimensional images of the inside of the body, for example starting from ultrasound images.

The fourth aspect relates to a diagnostics process not necessarily forming part of an IORT procedure or other radiotherapy procedure.

The fourth aspect relates to a diagnostic process comprising the following operations:
S.1bis) providing a system (1") comprising:
   - a diagnostics subsystem for images in turn comprising at least one probe 13;
   - a position detection subsystem (11);
S.2bis) by means of the at least one probe (13) acquiring a plurality of images (IM_1, IM_2, IM_i, IM_N) of internal sections of a body to be treated (P);
S.3bis) by means of the position detection subsystem (11) detecting the position in space of the probe (13) whilst it acquires each of said images (IM_1, IM_2, IM_i, IM_N);
S.5bis) by means of the diagnostics subsystem for images, deriving from the images (IM_1, IM_2, IM_i, IM_N) a three-dimensional model of the internal structure of at least a portion of the body to be treated (P).

Advantageously, the probe (13) is an ultrasound probe; preferably of the linear type; preferably it is designed to be gripped manually by a human operator, preferably with a single hand.

The fifth aspect relates to a diagnostic system (1") comprising:
- a diagnostics subsystem for images in turn comprising at least one probe 13;
- a position detection subsystem (11);
   and wherein:
- the at least one probe (13) is designed for acquiring a plurality of images (IM_1, IM_2, IM_i, IM_N) of internal sections of a body to be treated (P);
- the position detection subsystem (11) is programmed or in any case designed for detecting the position in space of the probe (13) whilst it acquires each of said images (IM_1, IM_2, IM_i, IM_N);
- the diagnostics system (1") is programmed or in any case designed for deriving from the images (IM_1, IM_2, IM_i, IM_N) a three-dimensional model of the internal structure of at least a portion of the body to be treated (P).

According to a sixth aspect, the present disclosure relates to a process for performing radiotherapy treatment, comprising the following operations:
S.1) providing a radiotherapy system (1) comprising:
   - a radiation head (3);
   - a movement system (7);
   - a diagnostics subsystem for images in turn comprising at least one probe 13;
   - a position detection subsystem (11);
S.2) by means of the at least one probe (13) acquiring a plurality of images (IM_1, IM_2, IM_i, IM_N) of internal sections of a body to be treated (P); S.3) by means of the position detection subsystem (11) detecting the position in space of the probe (13) whilst it acquires each of said images (IM_1, IM_2, IM_i, IM_N);
S.4) on the basis of the images (IM_1, IM_2, IM_i, IM_N) and by means of the movement system (7) moving the radiation head (3) and performing a predetermined treatment on the body to be treated (P).

According to a particular example of the process, the position detection subsystem (11) determines the position in space of the plurality of images (IM_1, IM_2 ... IM_i... IM_N) - preferably ultrasound images - and/or, if necessary, the above-mentioned virtual model of the inside of the body of the patient (P) according to the same reference system of linear and/or angular coordinates (X, Y, Z; α, β, γ; X', Y', Z'; α', β', γ'), according to which the movement system (7) and/or the position detection subsystem (11) determines the position and/or movements in space of the radiation head (3).

According to a particular example, the process comprises the operation of converting, by means of a suitable logic unit, the position in space of the plurality of images (IM_1, IM_2 ... IM_i... IM_N) - preferably ultrasound images - from the reference system (X, Y, Z, α, β, γ) originally used by the position detection subsystem (11) to the reference system (X', Y', Z', α', β', γ') originally used by the movement system (7).

According to a particular embodiment of this process, the position detection subsystem (11) comprises a distance detection system (120) and the process comprises the following operations:
- fixing at least one real position marker (110) to the at least one probe (13);
- detecting, for example in real time, the position and orientation in space of the at least one real position marker (110) by means of the distance detection system (120).

Further features of the invention are the object of the dependent claims.

The advantages which can be achieved with the invention are more apparent, to sector technicians, from the following detailed description of some particular embodiments of a non-limiting nature, illustrated with reference to the following schematic drawings.

### List of drawings

Figure 1 shows a perspective view of a particle accelerator of a radiotherapy system according to a first embodiment of the invention;
Figure 2 shows a first perspective view of a diagnostics subsystem for images and detection of the position of the radiotherapy system of Figure 1;
Figure 3 shows a second perspective view of a diagnostics subsystem for images and detection of the position of the radiotherapy system of Figure 1;
Figure 3A shows a side view of the manual probe and of the relative real position marker of the diagnostics system of Figure 3;
Figure 4 shows a perspective view of a detail of the radiation head of the particle accelerator of Figure 1;
Figure 5 shows a side view of the tubular applicator of the radiation head of Figure 4;
Figure 6 shows a side view of a second tubular applicator which may be mounted on the radiation head of Figure 4;
Figure 7 shows a perspective view of the arrangement in space of the ultrasound images obtained with the radiotherapy system of Figure 1;
Figure 8 shows a perspective view of a manual pointer of the radiotherapy system of Figure 1;
Figure 9 shows a perspective view of a diagnostics subsystem for images and detection of the position of the radiotherapy system according to a second embodiment of the invention;
Figure 10 shows a perspective view of a particle accelerator of a radiotherapy system according to a third embodiment of the invention;
Figure 11 shows a perspective view of a system for performing radiological treatments according to a fourth embodiment of the invention;
Figure 12 shows a side view of the system of Figure 11;
Figure 13 shows an image of a virtual model of a body to be treated acquired by means of the system of Figure 11;
Figure 14 shows a perspective view of a real position marker of the second type, belonging to the system of Figure 11;
Figure 15 shows a perspective view of a logic diagram for acquiring images of the body of a patient to be examined according to sagittal, coronal and transverse section planes or section planes parallel to them.

### Detailed description

The expression "radiological treatment" used in this description means a treatment of a body to be treated P by means of ionizing rays such as, for example, electromagnetic waves of extremely small wavelength, in particular X rays and/or γ [gamma] rays or in any case electromagnetic radiation with a wavelength equal to or less than 10 nanometres, electrons having an energy equal to or greater than 10 electron volts or corpuscular radiations originating, for example, from radioactive disintegrations.

This treatment may be of a therapeutic type and also non-therapeutic type, for example, exclusively cosmetic; it may be surgical and also non-surgical; diagnostic and also non-diagnostic; it may be used on a live human body, animal or vegetable, a dead human body, animal or vegetable or another inanimate object such as, for example, a mechanical, electrical or electronic component, a mineral or a semi-worked product.

The expression "radiological treatment" used in this description refers also to, but not necessarily, therapeutic, surgical or diagnostic treatments.

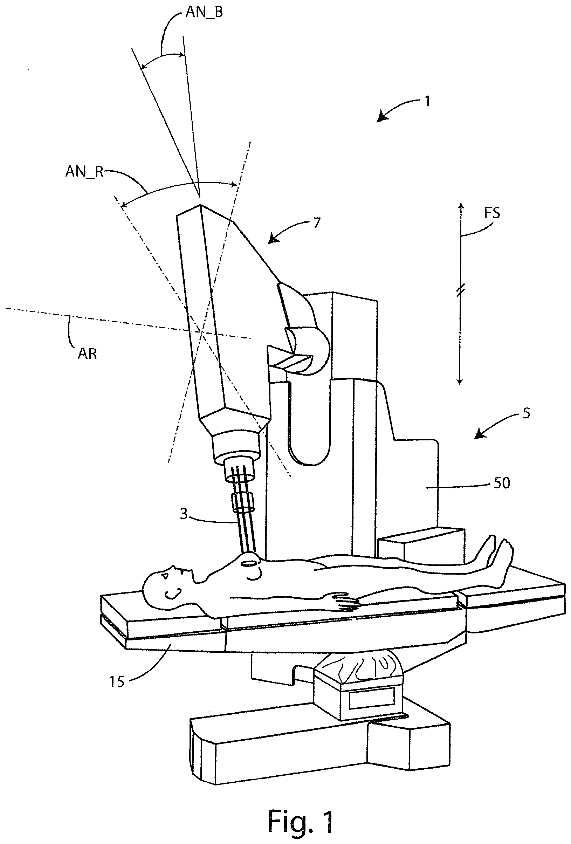

Figures 1-8 are relative to a system for performing intraoperative radiotherapy treatments according to a first embodiment of the invention.

The system is denoted in its entirety with reference numeral 1 and comprises:
- a radiation head 3;
- a movement system (7);
- a diagnostics subsystem for images in turn comprising at least one probe 13;
- a position detection subsystem 11.

The radiation head 3 is a component which is able to emit a radiation beam which can be used for therapeutic applications, such as, for example, a beam of electrons, photons, protons or ions.

The system 1 preferably comprises a suitable particle generator 5, for example a linear accelerator (LINAC, LINear ACcelerator) or non-linear accelerator, of known type, which generates the particles and accelerates them to a suitable energy to generate the beam which is then emitted - after being, if necessary, collimated or concentrated - from the radiation head 3.

The accelerator 5 can, for example, accelerate electrons to an energy of between 6-12 MeV (Mega electron volts).

The radiation head 3 can comprise, for example, an applicator 30, 30' having, for example, a tubular shape and made from suitable plastic material, for example polymethylmethacrylate (PMMA), having the aim of suitably shaping the radiation beam emitted by the source, which is of known type.

The tubular applicator 30, 30' can have, for example, one or more of the following features:
- an average internal diameter DT between 3-20 centimetres or between 3-12 centimetres;
- the maximum length LT between 20-120 centimetres or between 40-60 centimetres;
- a free end cut substantially at 90°or bevelled with an angle of, for example, 15°, 30°of 45°.

The free end is preferably designed for being inserted in the surgical incision.

The tubular applicator 30, 30' advantageously comprises an upstream section 300 and a downstream section 302, reversibly fixed to each other, for example by mans of a suitable quick coupling system.

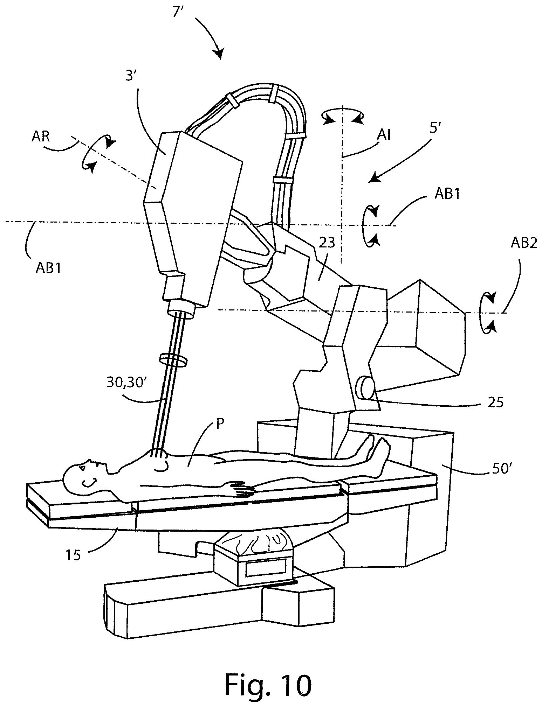

The movement system 7 is designed or moving and positioning - preferably in three-dimensional space - the radiation head 3 and in particular the relative tubular applicator 30, 30', and can comprise, for example, a right-angled mechanical manipulator, that is to say, Cartesian, or anthropomorphic (Figure 1, 10).

According to the embodiment of Figure 1, for example, the movement system 7 can comprise a mechanical manipulator with three degrees of freedom and which is able to make the radiation head 3 perform the following movements:
- raising and lowering it vertically, for example along the arrow FS;
- rotating the radiation head 3 about an axis AR, that is to say, tilting it by an angle AN_R - for example between 40°-80° - that is to say, executing rotations conventionally indicated, in this description, as "rolling rotations";
- rotating the radiation head 3 by an angle AN_B in the ideal plane in which lie the axis AR - conventionally indicated, in this description, as "rolling axis"
- and the axis of the tubular applicator 30, 30' of the radiation head or, more generally, the axis of the head 3, that is to say, executing rotations conventionally indicated, in this description, as "pitching rotations".

The linear accelerator 5, 5' preferably comprises a base 50, 50' designed for resting on an underlying paving or ground.

The base 50 is preferably equipped with wheels (not illustrated) which allow it to slide along the underlying paving.

If necessary, the movement of the wheels can be actuated by one or more motors and controlled with precision, for example, by means of position and/or speed sensors, in order to render them substantially as further controlled axes of a robot and render the base 50, 50' and the entire accelerator 5, 5' self-propelled.

The movement system 7 is preferably fixed to the base 50 and designed to move and position the radiation head 3 with respect to the base 50.

The diagnostics subsystem for images is, advantageously, an ultrasound system and comprises an ultrasound probe 13.

Preferably, the ultrasound probe 13 has dimensions e and shape such as to be able to be gripped by an operator, preferably with a single hand.

Preferably, the ultrasound probe 13 is of the linear type, that is to say, the piezoelectric crystals or other electronic or mechanical components which emit the ultrasounds are arranged along a segment which is substantially straight in length; the segment can have a length, for example, of between 5-30 centimetres, between 7-20 centimetres, between 8-12 centimetres or approximately equal to 10 centimetres.

A linear ultrasound probe 13 offers the advantages of generating images which are not distorted and which have a substantially rectangular or square shape.

Advantageously, the ultrasound probe 13 is equipped with pressure sensors designed fro measuring the pressure with which the probe is pressed on the scanned tissues.

Advantageously, the ultrasound probe 13 or, more generally, the diagnostics subsystem for images are designed to signal to the operator that the grip or in any case the use, if the probe 13 is pressed on the scanned tissues with a pressure equal to or greater than a predetermined threshold pressure, for example by emitting a visual or acoustic signal.

Advantageously, the predetermined threshold pressure has a sufficiently low value to prevent substantial deformations of the tissues scanned by the probe, and consequent deformations of the ultrasound images IM_1, IM_2 ... IM_i... IM_N which are acquired; so as to increase the precision of the ultrasound images and, therefore, of the resulting radiotherapy treatment.

The position detection subsystem 11 is designed to determine the position in space of the probe 13 and of the radiation head 3 according to a shared reference system (X, Y, Z; α, β, γ) or (X', Y', Z', α', β', γ').

Preferably, the position detection subsystem 11 is designed to determine the position in space according to three Cartesian axes XYZ or, in any case, not coplanar, and the orientation in space with three angles α [alfa], β [beta], γ [gamma] referred to three angular reference positions.

For example, the three angles α [alfa], β [beta], γ [gamma] can indicate the inclinations of the probe 13 and of the radiation head 3 with respect the three axes XYZ or to the three planes XY, YZ, XZ.

Again for this purpose, the position detection subsystem 11 can comprise one or more real position markers 110 and a remote tracking system designed for remotely determining the position in space.

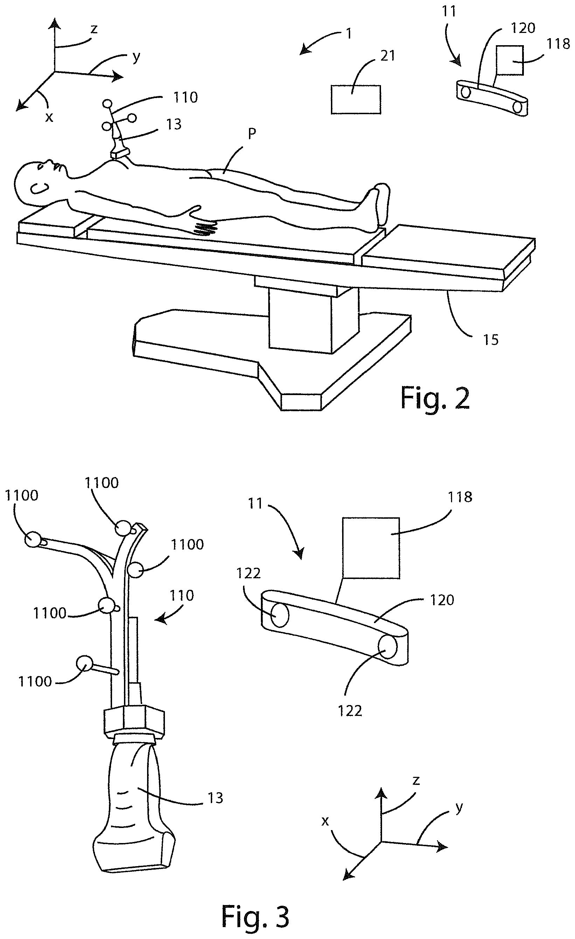

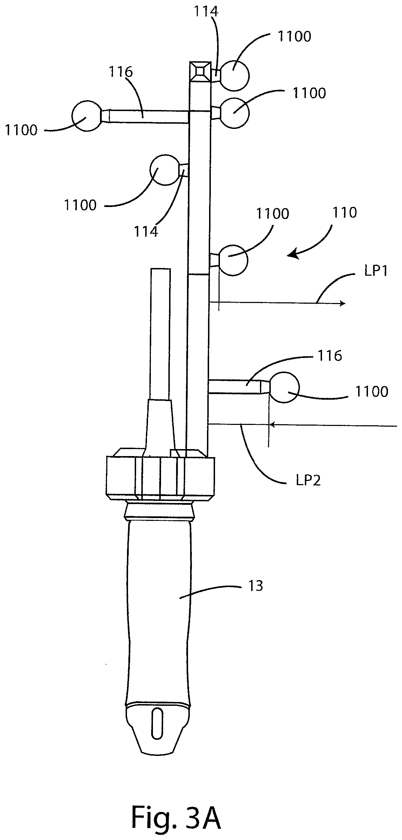

In accordance with the embodiment of Figures 2, 3 each real position marker can comprise one or more spheres 1100, balls or other objects which are substantially point-like or in any case with have much smaller dimensions than the real object to which it is applied and of which it must determine the position, these objects facilitating the recognition by, for example, an optical or remote electromagnetic system.

Alternatively, each real position marker 110 can also comprise one or more objects which are not "point-like" such as, for example, rods and bars or lines or other marks drawn, printed or in any case indicated on a transparent or opaque wall.

Preferably, in accordance with the embodiments of Figures 2, 3 each real position marker 110 comprises a plurality of bodies which are substantially point-like, globular or rounded such as, for example, at least six balls 1100 which are not coplanar with each other, so as to be able to identify all six degrees of freedom of a rigid body with finite dimensions in space.

In accordance with the preferred embodiments of Figures 3, 3A, 4, 8, each real position marker 110, 110', 110" can comprise a frame which in turn comprises a portion of frame 112 having a substantially "Y" or fork-like shape, and a plurality of pins 114, 116 which protrude from the fork-like frame 112 and at the free ends of which are fixed the balls or other globular or rounded bodies 1100.

More specifically, the fork-like frame 112 advantageously has a substantially planar shape, that is to say, it lies substantially on a plane.

Advantageously, the plurality of pins 114, 116 protrudes from the fork-like frame 112 and extend in directions substantially perpendicular or transversal to the plan on which the fork-like frame 112 lies.

Advantageously, at least two of the at least six balls or other globular or rounded bodies are positioned on the same larger face of the fork-like frame 112.

Advantageously, the frame of a real position marker 110, 110', 110" comprises at least two pins 116 having length LP2 greater than the length LP1 of the other pins 114; for example, the ratio LP2/PL1 is preferably equal to or greater than 4 times, more preferably equal to or greater than 6 times and even more preferably equal to or greater than 8 times.

Advantageously, at least one long pin 116 protrudes from each larger face of the fork-like frame 112.

In this way, balls or other rounded bodies 1100 can be easily seen and recognised by an optical tracking system, whatever the position and orientation in space of the probe 13, the applicator 3 or the pointer 17 to which the relative marker 110, 110', 110" is fixed, with the reduction in the errors in detection of the position, orientation and distance of the marker.

According to an embodiment not illustrated, a real position marker can comprise, for example, a polyhedron on the vertices of which can, if necessary, be present balls, other objects which are substantially point-like, globular or rounded.

In accordance with the embodiment of Figure 1, the remote tracking system advantageously comprises a logic unit 118 programmed or in any case designed for detecting the distance, position and orientation in space of the real position markers 110.

In order to do this, the logic unit 118 can be programmed or in any case designed for acquiring the images of the real markers 110 or remotely determining the position by detecting suitable electric or magnetic fields, for example in the case in which each ball 1100 or rod emits electromagnetic waves which are not necessarily visible, or acoustic waves.

The logic unit 118 can be programmed or in any case designed for acquiring the images of the real markers 110 for example in the visible light, infrared or ultraviolet band.

For this purpose, the logic unit 118 can be programmed and run a suitable program for image recognition and optical or positional tracking.

Again for this purpose, the remote tracking system can comprise a suitable stereoscopic camera or video camera 120 which generates and send the images - static or video - to the logic unit 118.

The stereoscopic camera or video camera 120 can in turn be equipped with two or more lenses 122 designed for generating stereoscopic or three-dimensional images.

As shown in Figures 1-4, advantageously on the probe 13 and on the particle accelerator 5, for example on the relative radiation head 3 is fixed and integral at least a respective real position marker 110, 110' equipped with at least six balls - respectively 1100, 1100' - or other substantially globular point-like bodies or six rods or lines which are not coplanar with each other in such a way as to allow the logic unit 118 to detect and remotely determine the position in the three linear coordinates (X, Y, Z) and in the three angular coordinates (α, β, γ) - corresponding to the three inclinations in space - of the probe 13 and of the radiation head 3, preferably of the relative tubular applicator 30.

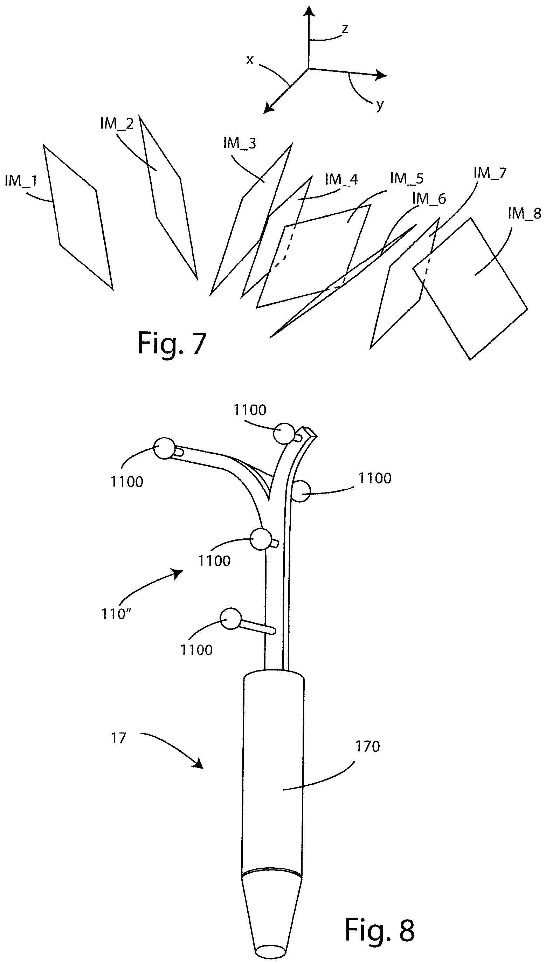

If necessary, the system 1 can comprise one or more pointers 17 designed to draw, mark or simply point to zones of particular interest about the surgical incision of the patient P and more in general zones of the relative body (Figure 8).

Each pointer 17 can comprise, for example, a pencil, pen or marker pen designed to make marks on the body of the patient, a luminous or laser stylus or marker 170 designed for projecting a luminous mark on the body of the patient.

Each pointer 17 is designed for allowing the position detection subsystem 11 to detect the position in space in terms of linear and angular coordinates.

For this purpose, each pointer 17 can be equipped with a relative real position marker 110", for example of the types described above.

The marker 110" is preferably fixed integrally with the portion of the pointer 17 which forms the pencil, pen, maker pen, stylus or optical pointer.

In accordance with the embodiment of Figure 8, the real position marker 110" of the pointer 17 is equipped with six balls 1100 not coplanar with each other and designed to be detected by the above-mentioned remote tracking system.

A particular example of operation and use is described below of the system 1 described previously.

The following description refers to a human patient P but it can clearly be adapted to an animal patient, an inanimate object such as, for example, an industrial product or other body to be treated.

The human patient P lies, for example supine and, if necessary, under a general anaesthetic, on the operating bed 15 after a tumour in the intestines, rectum or pancreas has been removed; the patient may still be in the operating room and on the same operating bed 15 on which the tumour has already been removed.

Preferably, the movements of the various limbs of the patient P are prevented by means of suitable immobilising devices with sufficient stiffness to allow, for example, successful performance of magnetic resonance (RM) or a computed axial tomography (TAC).

In other words. the patient P can be blocked by suitable immobilising devices fixed to the operating bed 15.

Advantageously, a fourth real position marker 110A is positioned on the operating bed 15.

The zone of tissues adjacent to the tumour and at greatest risk of relapse, that is to say, the tumour bed, are now to be subjected to localised radiotherapy.

The surgical incision through which the tumour has been removed is still, for example, open.

Advantageously, a radiologist, other doctor or human operator grips the pointer 17 and, using it, draws or simply indicates areas of particular medical interest, for example encircling or in any case enclosing with one or more real or merely virtual boundary marks the space, of the body of the patient P, to be acquired with the ultrasound probe 13, or marking with real or merely virtual marks the zone of removal of the tumour or any temporary sutures.

The position detection subsystem 11 detects and acquires the positions, orientations and trajectories in space of the pointer 17.

A radiologist or other human operator gripping the ultrasound probe 13 performs a manual scanning of the zone of the surgical incision, acquiring in particular one and preferably more ultrasound images IM_1, IM_2 ... IM_i... IM_N of the incision and of the adjacent organic tissues to be irradiated.

These images can be, for example, digital images.

As shown in Figure 2 the radiologist preferably grips the ultrasound probe 13 in such a way that each ultrasound image IM_1, IM_2 ... IM_i... IM_N acquired is a section along an ideal plane which penetrates inside the body of the patient, for example according to a plane approximately coincident with or parallel to the coronal, transverse or sagittal plane of the patient P.

The above-mentioned pressure sensors of the probe signal to the operator whether the tissue to be scanned is being pressed too much, preventing its deformation, and consequently the deformation of the ultrasound images.

With the system 1 and relative process, even though advantageous, it is not absolutely essential that the planes of the various ultrasound images are precisely parallel or equidistant to each other; as explained more clearly below, the planes of the various ultrasound images can be inclined even by a few tens of degrees with respect to the adjacent ones.

However, the operator can, for example, acquire a plurality of ultrasound images IM_1, IM_2 ... IM_i... IM_N which lie on planes more or less alongside and approximately parallel with each other, as shown, for example, in Figure 7.

Advantageously, every time an ultrasound image IM_1, IM_2 ... IM_i... IM_N is acquired, the remote tracking system, for example the camera or video camera 120 films the six balls 1100 of the real position marker and determines the position of the balls in space in terms of linear coordinates, according to the relative reference system (X, Y, Z).

The reference system (X, Y, Z) can be, for example, the "native" one of the position detection subsystem 11, that is to say, the one in which the system 11 originally determines the position of the real marker 110, 110', 110" or of other objects in general.

From the positions of the six balls 1100 the remote tracking system determines the position in space, in terms of linear and angular coordinate in the three-dimensional space, of the real position marker 110 fixed on the probe 13 and from this it can then determine the position in space of each ultrasound image IM_1, IM_2 ... IM_i... IM_N as they are gradually acquired.

More specifically, the remote tracking system preferably determines the position in space, in terms of linear and angular coordinates in space, of each ultrasound image IM_1, IM_2 ... IM_i... IM_N.

The remote tracking system preferably determines and associates three linear coordinates (x_i, y_i, Z_i) and three angular coordinates (α_i, β_i, γ_i) to each ultrasound image IM_i thereby uniquely identifying their position in three-dimensional space.

Having, in a virtual fashion, the various ultrasound images IM_1, IM_2 ... IM_i... IM_N in the three-dimensional space, the logic unit 118 or other logic unit of the system 1 can reconstruct, for example, a virtual three-dimensional model of the inside of the zone of the body of the patient P undergoing ultrasound examination; the position in space of this virtual model being known as the position is known of the various ultrasound images IM_1, IM_2 ... IM_i... IM_N.

Advantageously, the position detection subsystem 11 determines, for example by means of the logic unit 118 or other logic unit, the position in space of the various ultrasound images IM_1, IM_2 ... IM_i... IM_N and of the above-mentioned virtual model of the inside of the body of the patient P according to the same reference system (X, Y, Z) or (X', Y', Z') used by the particle accelerator 5 for controlling and commanding the position of the radiation head 3 and the movements of the movement system 7.

For this purpose, the remote tracking system can detect, for example by means of the camera or video camera 120 or other camera or video camera, the position in three-dimensional space of the real position marker 110' fixed integrally to the radiation head 3, the tubular applicator 30; in this case, preferably, the remote tracking system detects or in any case determines the position of the marker 110' by means of three linear coordinates X, Y, Z and three angular coordinates α, β, γ.

The angular coordinates indicate the three inclinations of the real position marker 110' with respect to the reference axes or planes in space.

Alternatively, the remote tracking system can detect, for example by means of the camera or video camera 120 or other camera or video camera, the position in three-dimensional space of the real position marker 110' fixed integrally to the base 50 of the particle accelerator 5, and from the position of the base 50 obtain the position of the radiation head 3 by means of the internal information of the movement system 7: in fact, in order to move and position with precision the radiation head 3, the movement system 7 knows the position in space with respect to the base 50 or other reference zone of the accelerator 5.

In the latter case, the position detection subsystem 11, by means of a suitable logic unit, such as, for example, the unit 118, transforms the position of the radiation head 3 according to the original reference system (X', Y', Z'; α', β', γ') into the reference system (X, Y, Z; α, β, γ) the position detection subsystem 11 of which detects - for example originally - the position of the real marker 110; or vice versa it can convert the position of the real marker 110 according to the relative native reference system (X, Y, Z; α, β, γ) into the native reference system (X', Y', Z'; α', β', γ') of the movement system 7.

In this way, thanks to the fourth real position markers 110, 110' and 110A, the system 1 can determine, for example in real time, the position in space of each ultrasound image IM_1, IM_2 ... IM_i... IM_N and therefore of the patient P and of the radiation head 3 in the same spatial reference system (X, Y, Z; α, β, γ) or (X', Y', Z'; α', β', γ').

In other words, providing that on the operating bed 15 is positioned the above-mentioned fourth real position marker 110A, or providing the patient P is not moved in the operating room in which he/she is located or more in general with respect to the particle accelerator or other radiation generator 5, the system 1 and in particular the logic unit 118 or other logic unit, for example the one which controls the movement system 7, is able to detect or in any case know at every instant the relative position in space of the patient P with respect to the radiation head 3, and is therefore able to control the movements of the latter and to position it on the body of the patient with a much greater precision with respect to that permitted by the currently known IORT systems and processes, substantially with the precision of a numerical control machine.

An ultrasound image IM_1, IM_2 ... IM_i... IM_N is substantially a series of pixels which lie in a plane in the three-dimensional space, but results from the exploration, by the probe 13, of a region of three-dimensional space, approximately with the shape of a relatively flat parallelepiped; for example, an image generated by an ultrasound probe 13 of linear type with a row of 10-centimetre long ultrasound emitters, approximately having the shape of a parallelepiped with a rectangular base, with a width of approximately 10 centimetres (corresponding to the penetration depth of the ultrasounds in the body of the patient) or between 7 and 15 centimetres or between 7 and 10 centimetre and thickness of approximately 2-3 centimetres.

For this reason, in order to obtain a particularly precise three-dimensional model of the inside of the body of the patient, one could explore with the probe 13 the entire space underlying the surface of the body of the patient P surrounded or enclosed by the above-mentioned one or more boundary marks.

For this purpose, one could consider exploring every point of the space to be explored with the acquisition of at least one ultrasound image.

The source 1 can be programmed or in any case designed for displaying on a screen a two-dimensional or three-dimensional map of the portions of the space already explored or still to be explored with the ultrasound probe.

The system 1 can also be programmed or in any case designed for emitting acoustic and/or visual alarm signals, for warning the operator when the ultrasound acquisitions for generating the three-dimensional model of the patient P have been completed, without having to completely explored the space to be explored.

On the basis of the three-dimensional model obtained from the ultrasound images, acquired preferably in the operating room, the radiotherapist or other doctor or operator can plan the radiotherapy treatment very accurately, for example by means of numerical simulations.

In fact, the three-dimensional ultrasound model of the inside of the patient P allows, for example:
- knowing with greater precision the structure, shape, dimensions and position of the target to be irradiated and of the adjacent healthy tissues and organs to be irradiated as little as possible;
- positioning in a virtual fashion various applicators 30, 30' on the images acquired, with a more weighted and carefully studied selection;
- calculating with greater precision with respect to the current systems the actual dose of radiation necessary; in particular, calculating the actual dose of radiation, as a function of the energy selected, on each point of the image or images acquired IM_1, IM_2 ... IM_i... IM_N.;
- simulating and performing a treatment also using two or more different applicators 30 and/or two or more different energies;
- acquiring and calculating, that is to say, simulating, the distribution of doses in the presence of beam modifiers such as, for example bolus and formers.

If necessary, the real or even only virtual marks previously traced by the pointer 17 on the body of the patient P can be added or viewed in the virtual three-dimensional model - for example on the screen of a workstation or other computer.

Advantageously, the three-dimensional model of the patient obtained from the ultrasound images IM_1, IM_2 ... IM_i... IM_N can be divided - by means of a suitable logic unit 21 - into small elementary spaces, for example into voxels with, for example, a shape and dimensions equal to each other, allowing the calculation with a greater precision of the necessary dose of radiation.

After selecting the applicator and the dose of radiation, the movement system 7 positions the radiation head 3 on the target on or in the body of the patent P - for example inserting the end of the tubular applicator 30, 30' in the surgical incision - and administering the requested dose of radiation.

In order to do this, the movement system 7 can be advantageously controlled automatically and with great precision from a suitable logic unit, for example the one inside the particle accelerator or other radiation generator 5, or from the logic unit 118.

In this way there is a greater certainty in positioning the radiation head 3 on the correct target, reducing, if not eliminating, the risks of imprecise positioning - especially with regard to the orientation in space of the applicator 30, 30' which, as already mentioned, influences considerably the dose of radiation received by the patient - and therefore on an ineffective treatment.

When the movement system 7 automatically positions the radiation head 3 on the target, it advantageously moves the applicator 30, 30' already mounted and complete for example for its upstream 300 and downstream 302 section.

Alternatively, the downstream section 302 of the applicator can be positioned manually in the surgical incision or in any case on the target, arranging it precisely in the position determined by means of the numerical simulation on the virtual three-dimensional model of the patient P obtained from the ultrasound images IM_1, IM_2 ... IM_i... IM_N.

For this purpose, the downstream section 302 can be positioned with precision in the surgical incision or in any case on the target fixing a real position marker 110' on the downstream section 302, and then checking in real time by means of the position detection subsystem 11 whether the downstream section 302 has been positioned in the optimum position determined previously with the three-dimensional model and the numerical simulation.

Once placed in the optimum position, the downstream section 302 can be fixed and kept in position by blocking it, for example, with a special frame which rests on the floor of the operating room or is fixed to the operating bed 15.

If the position detection subsystem 11 comprises the second mechanical arm 19, the latter can place the downstream section 302 in the surgical incision or on another target with the optimum position and orientation in space determined previously with the three-dimensional model and the numerical simulation.

After this, the movement system 7, guided by a human operator for example by means of a suitable remote control unit or guided by a suitable logic unit, moves the radiation head in such a way as to couple the upstream 300 and downstream 302 sections of the applicator 30.

A great advantage of the system 1 and of the process for using it described previously is the possibility of performing simulations of the radiotherapy treatment when the patient is on the operating table during the surgical operation, acquiring a three-dimensional model of the inside of the patient and the relative position in space in a very fast and convenient manner - the model can in fact be obtained using the manual probe 13 - without the need to move or shift the patient in order, for example, to introduce it in a magnetic resonance or axial tomography machine.

In particular, the system 1 makes it possible to keep the patient P perfectly still from the start of the surgical operation and/or radiotherapy - for example for removing a tumour - until completion of the radiotherapy treatment, in particular without having to remove and reapply any immobilising devices which keep the patient in position, unlike what is necessary, on the other hand, for introducing the patient, for example, in a magnetic resonance or axial tomography machine.

Clearly, the above-mentioned virtual model of the inside of the patient
- or at least of the zone of the body to be undergo radiotherapy - can be improved and enriched with the necessary densitometric information, depending on the clinical cases.

Generally, due to the specific nature of the IORT treatment, the target tissue of the irradiation is never significantly different from the water/tissue equivalent (for example, bone, tendons and lungs are generally not to be irradiated); it can therefore be reasonably assumed that the density of the image acquired is that of water.

If necessary, a fusion of images between a pre-op ultrasound scanning and a pre-op CT may be performed; in this way the corresponding Hounsfield number from the computed tomography (CT) is associated with each "voxel" of the ultrasound model and this information is stored for reuse in the post-op scanning.

If necessary, it is also possible to perform the post-op scanning by inserting materials with a known geometry and chemical composition, for example, a 1 mm sheet of PMMA or other bolus with known density and thickness to be positioned above the tissue, that is to say, the radio-protective disk in the case of treatment of the mammary carcinoma.

The system 1 described previously, in particular the relative ultrasound probe 13, results in very low purchase and management costs, is very simple to use and allows intraoperative radiotherapy to be performed even by medical personnel who are not highly skilled on anatomic districts which are currently considered to be difficult and in hospitals which are not centres of excellence; it also allows imaging techniques to be used during intraoperative radiotherapy.

The preparation of the system in the operating room, the acquisition of the ultrasound images and the generation of the three-dimensional model of the inside of the patient is very fast and can be performed in less than 5 minutes.

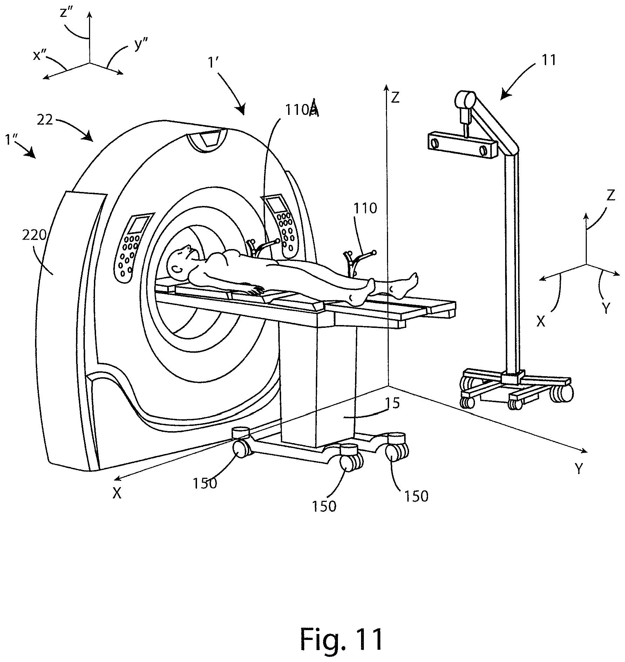

Figures 11-14 are relative to a system and a process for performing radiological treatments, for example intraoperative radiotherapy, according to a fourth embodiment of the invention.

The system, denoted in its entirety with reference numeral 1", comprises a radiological treatment system in turn comprising the above-mentioned radiation head 3, the above-mentioned movement system 7, a diagnostics subsystem for images 22 and a position detection subsystem 11.

The diagnostics subsystem for images 22 is designed for acquiring or generating a virtual model of the inside of the above-mentioned body to be treated P.

The model may comprise, for example, one or more two-dimensional images IM_1...IM_n or directly a three-dimensional model - for example numerical - of the inside of the body to be treated P.

The virtual model can be analogue or digital; it can have the form of an electronic document, for example a data file, or a hard copy document or a two-dimensional or three-dimensional object.

For this purpose, the diagnostics subsystem for images 22 may comprise, for example, one or more of the following systems: a scanner for providing magnetic resonance, computed axial tomography, radiological stratigraphy, positron emission or single-photon emission computed tomography, ultrasound, Doppler ultrasound, radiography, fluoroscopy, angiography, scintigraphy images.

More specifically, the diagnostics subsystem for images 22 may comprise, for example, a scanner 220 for acquiring images or virtual models by magnetic resonance, computed axial tomography, radiological stratigraphy, positron emission or single-photon emission computed tomography, ultrasound, Doppler ultrasound, radiography, fluoroscopy, angiography, scintigraphy.

The position detection subsystem 11 can be, for example, of the types described previously with reference to Figures 1-10.

Advantageously, the system 1" also comprises a logic unit 118 programmed or in any case designed for determining the position in space of said virtual model (IM_1, IM_2, IM_i, IM_N) and of the radiation head 3 according to a same reference system of linear and/or angular coordinates (X, Y, Z; α, β, γ; X', Y', Z'; α', β', γ'; X", Y", Z"; α", β", γ").

Advantageously, the system 1" is programmed or in any case designed for moving the radiation head 3 on the basis of said virtual model IM_1...IM_n and by means of the movement system 7.

For example, on the basis of the virtual model IM_1...IM_n and by means of the movement system 7 the system 1" can be programmed or in any case designed to place the radiation head 3 at or close to a zone of the body P which constitutes a target to be irradiated, for example a surgical incision made in the body of a patient P to be treated, where the body P to be treated may be the body of a human being, an animal or a vegetable from which a tumour has been previously removed.

The scanner 220 of the diagnostic system for images can form an internal tunnel 2200 designed to house partly or completely the body of a patient or other body to be treated P.

Advantageously, the system 1" comprises the operating bed 15, preferably equipped with wheels or runners so that it slides on a floor, for example of an operating room.

The operating bed 15 can be replaced by a more generic treatment support 15 designed for supporting and positioning a patient or another body to be supported P, for example a human patient or animal immobilised and fixed on the treatment support 15.

Advantageously, the operating bed or other treatment support 15 is equipped with at least one real position marker of a first type 110 and at least one real position marker of a second type 110A, where the real position marker of the first type 110 is designed to be detected at least by the position detection subsystem 11, for example by a remote tracking system comprising a stereoscopic camera or video camera 120 sensitive to visible light, whilst the real position marker of the second type 110A is designed to be detected at least - and preferably - also by the diagnostics subsystem for images, for example by a magnetic resonance scanner or by computed axial tomography with X-rays, positron emission or single-photon emission, an ultrasound scanner if necessary for Doppler ultrasounds; for this purpose, the real position marker of the second type 110A can be made from a suitable polymeric material.

If the diagnostic system for images comprises an X-ray receiver, the real position marker of the second type 110A is made of a suitable radio-opaque material.

If the diagnostic system for images comprises a magnetic resonance scanner, the real position marker of the second type 110A can be made, for example, of aluminium or another suitable non-ferromagnetic material.

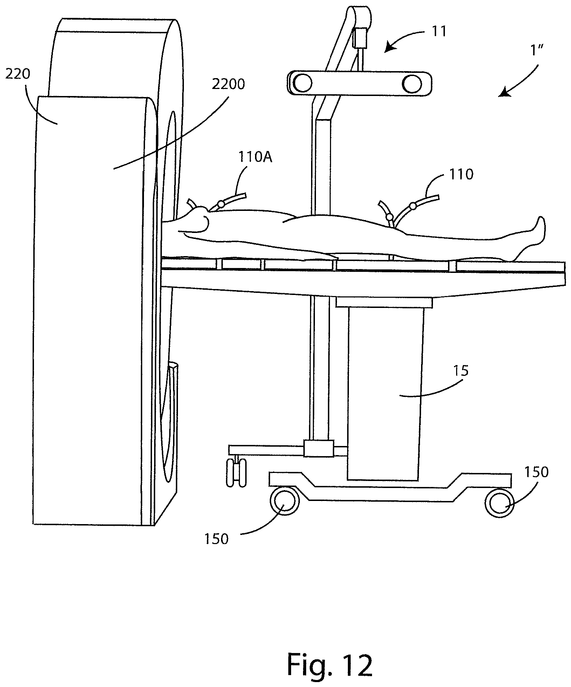

As, for example, in the embodiments of Figures 11, 12, the real position markers of the first type 110 and of the second type 110A can both be fixed to the operating bed or other treatment support 15.

Each of the markers 110, 110A can have the shape of the markers 110, 110', 110" described above and comprise, for example, one or more spheres 1100, balls or other objects which are substantially point-like or in any case with have much smaller dimensions than the real object to which it is applied and of which it must determine the position, these objects facilitating the recognition by, for example, an optical or remote electromagnetic system.

Alternatively, each real position marker 110, 110A can also comprise one or more objects which are not "point-like" such as, for example, rods and bars or lines or other marks drawn, printed or in any case indicated on a transparent or opaque wall.

Preferably, according to the embodiment of Figures 11, 12 each real position marker of the first type 110 and of the second type 110A comprises a plurality di substantially point-like, globular or rounded bodies such as, for example, at least six balls 1100, 1100A which are not coplanar with each other, so as to be able to identify all six degrees of freedom of a rigid body with finite dimensions in space, where the bodies 110 can be, for example, visible to the stereoscopic camera or video camera 120 operating in the visible light band, whilst the bodies 110A can be visible, for example, from a magnetic resonance scanner 220 or from the other above-mentioned diagnostic methods for images.

In accordance with the preferred embodiments of Figures 3, 3A, 4, 8, each real position marker 110, 110A can comprise a frame which in turn comprises a portion of frame 112 having a substantially "Y" or fork-like shape, and a plurality of pins 114, 116 which protrude from the fork-like frame 112 and at the free ends of which are fixed the balls or other globular or rounded bodies 1100, 1100A.

More specifically, the fork-like frame 112 advantageously has a substantially planar shape, that is to say, it lies substantially on a plane.

A particular example of operation and use is described below of the system 1" described previously.

In a same operating room there is the scanner 220 of the diagnostics system for images 22, the remote optical tracking system - that is to say, at least in the visible light band - and the relative stereoscopic camera or video camera 120, the operating bed 15 and the particle accelerator 5 which must perform the radiotherapy treatment on the patient P or other radiological treatment on another type of body to be treated P, for example a mechanical component or a prostheses.

On the operating bed 15 are fixed integrally, for example, a real position marker of the first type 110 and another marker of the second type 110A, both having a shape, for example, similar to that of Figure 8.

The scanner 220 of the diagnostics subsystem for images 22 may be, for example, a magnetic resonance scanner.

On the operating bed 15 is placed a patient P preferably immobilised, for example with suitable immobilising devices such as to allow the successful performance of a magnetic resonance (RM) or a computed axial tomography (TAC) or in any case the pre-selected scanning.

For this reason, the relative position of the patient or other body to be treated P with respect to the markers 110, 110A does not vary during the scanning.

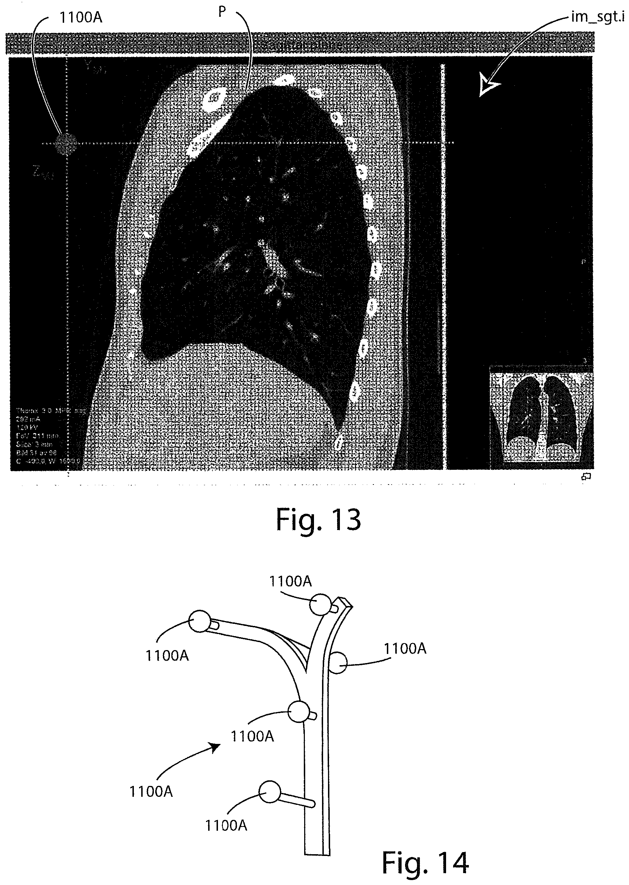

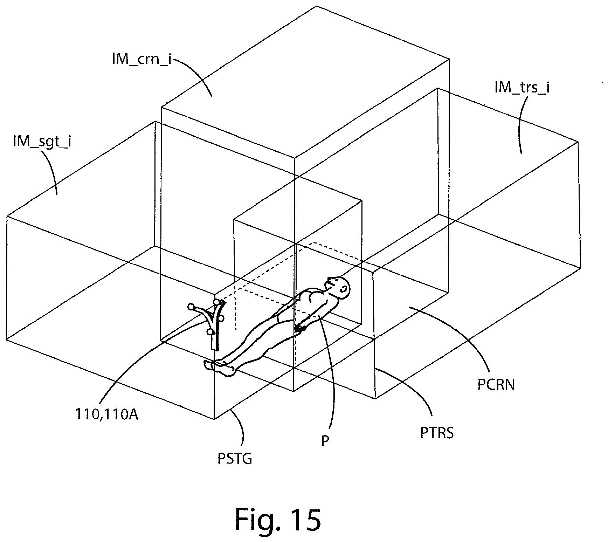

The operating bed 15 is moved in the operating room so that it slides, for example, on its wheels 150, and in that way the body to be treated P is, for example, introduced in the tunnel 2200 of the scanner for acquiring a scan, generating one or more images such as that of Figure 13. Together with a section of the body P - for example according to a sagittal section plane PSGT of the body P to be treated or according to a plane parallel to it - the image also shows a section of the real position marker of the second type 110A, in its precise linear and angular position in space with respect to the body to be treated P; in a parallel direction, the diagnostic system for images 22 can simultaneously acquire or generate the virtual model - for example numerical - both of the portion of body to be treated P and of the real position marker of the second type 110A and of the relative position of the latter in three-dimensional space with respect to the body P to be treated resting on the bed 15; for this purpose, the diagnostic system for images 22 can, for example, acquire several images IM_crn.i, IM_trs.i similar to that of Figure 13 but which show sections according to ideal planes parallel not only to the sagittal plane PSGT, but also the coronal plane PCRN and the transverse plane PTRS of the body P to be treated, and, between these images in three different section planes in space, obtain the virtual model of the body to be treated P and of the real position marker of the second type 110A (Figure 15).

From this virtual model, preferably if numerical or in any case in an electronic format, the logic unit 118 determines the position in three-dimensional space and the position of the real position marker of the second type 110A according to a shared system of linear and/or angular reference coordinates (X", Y", Z"; α", β", γ") relative to the scanner 220.

Firstly, whilst or after the scanner 220 has acquired the virtual model of the body P to be treated, the position detection subsystem 11 detects the linear and angular position in space of the real position marker of the first type 110 fixed to the operating bed 15, in a second linear and angular reference system (X, Y, Z; α, β, γ) relative to the position detection subsystem 11.

The two reference systems (X", Y", Z"; α", β", γ") of the diagnostics subsystem for images 22 and (X, Y, Z; α, β, γ) of the position detection subsystem 11 can therefore be correlated, for example by means of the logic unit 118 or another unit, obtaining the position of one with respect to the other, for example knowing the linear and angular position of each of the two real position markers 110, 110A with respect to the other.

Once the magnetic resonance, the computed axial tomography or other detection of the diagnostics subsystem for images has been acquired, the patient or other body to be treated P can be extracted from the scanner 220 and moved towards, for example, the radiation head 3 or, more in general, the particle accelerator 5.

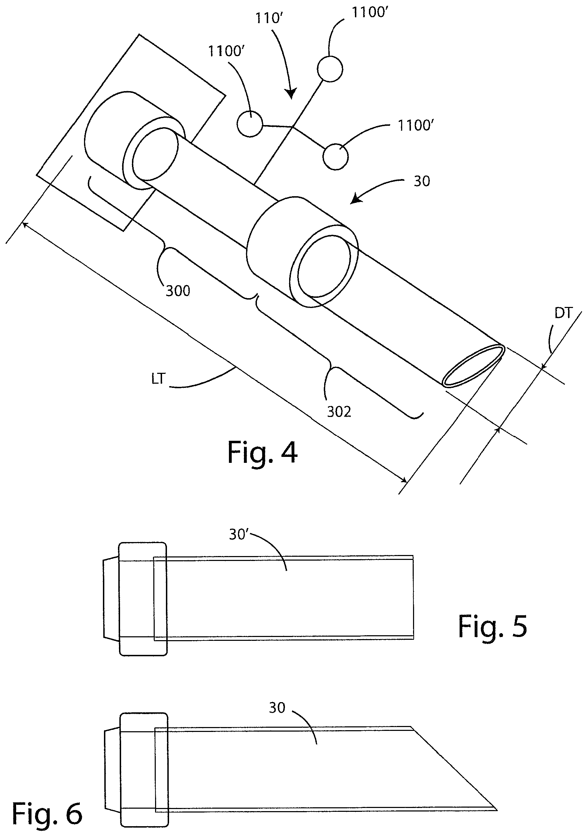

As described above, with reference to Figures 1-10, the position detection subsystem 11 also determines, for example by means of the stereoscopic camera or video camera 120 and relative optical tracking software, the position in space, according to the relative (third) linear and angular reference system (X, Y, Z; α, β, γ), of the radiation head 3, for example detecting the position of the real position marker of the first type 110' fixed on it (Figure 4): this allows, as already described, correlation of the two linear and angular reference systems (X, Y, Z; α, β, γ) of the position detection subsystem 11 and (X', Y', Z'; α', β', γ') of the radiation head 3 and relative movement system 7.

Consequently, the logic unit 118 or other unit can now correlate, establishing the positions in space of one with respect to the others, the three linear and angular reference systems of the scanner 220, of the position detection subsystem 11 and of the radiation head 3 (with relative movement system 7).

The logic unit 118 can also determine the linear and angular position in space of the virtual model (IM_1...IM_n) of the inside of the body P according to any of the three above-mentioned reference systems.

The movement system 7 can now move, automatically and with considerable precision, the radiation head 3 in space, arranging it in the desired position, for example close to or inside a surgical incision in which there is a tumour bed to be irradiated or, more simply, close to or inside a mechanical part to be treated P.

The radiation head 3 can therefore irradiate with greater precision - for example, because it is positioned in space with greater precision - a dose of radiation on the target, after the system 1" has calculated it with greater precision on the basis of the virtual model (IM_1...IM_n) of the inside of the body P and its position in space; this position being determined (also) according to the relative linear and angular reference system.

Advantageously, the real position marker or markers of the second type 110A are such that - that is to say, they have embodiments and are made of materials such that - they can be detected both by the scanner 220 of the diagnostics subsystem for images 22 and by the optical tracking system or other position detection subsystem 11.

This allows a single real position marker 110A, either of the first or second type, to be fixed on the operating bed 15; the scanner 220 and the optical tracking system or other position detection subsystem 11 detects the position in space of the same real position marker 110A for determining the position in space of the virtual model (IM_1...IM_n) of the inside of the body P, as it is not necessary to obtain the position of the real position marker of the second type 110A from the position of a real marker of the first type 110, thereby reducing the errors in determining the positions.

The embodiments described above can be modified and adapted in several ways without thereby departing from the scope of the inventive concept.

For example, the real position marker or markers 110' can be fixed not only on the radiation head 3 but, for example, also on the base 50 of the linear accelerator or other particle or radiation generator 5; in that case, the position detection subsystem - for example a stereoscopic, optical or remote system - can determine, for example in real time, the position of the radiation head 3 and in particular of the free end of the tubular applicator 30, 30', as well as the position of the marker 110', from the movements of the controlled axes of the movement system 7, from example from the encoders or other position transducers of the kinematic mechanisms of the movement system 7.

The position detection subsystem can also comprise distance detection systems using radar with electromagnetic and/or acoustic waves or laser pulse radar (LIDAR).

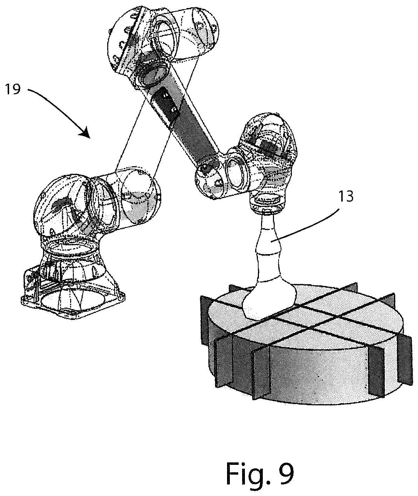

The position detection subsystem may also not be based on a stereoscopic or optical or remote system, such as subsystem 11, and can comprise, for example, a second mechanical arm 19, which can be, for example, an anthropomorphic arm (Figure 9).

The ultrasound probe 13 can be fixed to the mechanical arm 19 - for example to its wrist - which positions the probe 13 and moves it during the scanning of the patient and the acquisition of the ultrasound images IM_1, IM_2 ... IM_i... IM_N.

Every time an ultrasound image IM_i is acquired, the second mechanical arm 19 detects the position in space, for example, as already mentioned, in terms of linear and angular coordinates in space according to the Cartesian reference system (X, Y, Z; α, β, γ) or native polar reference system of the arm 19.

For example, the positions in space of the ultrasound images can be obtained from the encoders or other position transducers present in the articulations of the mechanical arm 19.

Once the three-dimensional model of the scanned anatomic zone of the patient P has been derived, the second mechanical arm 19 is used for moving the radiation head 3, for example fitting it to the wrist of the arm 19, and positioning it as required by the planned radiotherapy.

Clearly, in order to move the radiation head 3 the mechanical arm refers preferably to the relative Cartesian reference system (X, Y, Z; α, β, γ) or native polar reference system, that is to say, the same one used for manipulating the probe 13 and determining the positions in space of the ultrasound images IM_1, IM_2 ... IM_i... IM_N.

The mechanical arm 19 is therefore able to move and position the radiation head 3 with considerable precision, for example equal to that of a numerical control machine.

According to the embodiment of Figure 10, for example, the movement system 7' can comprise an anthropomorphic mechanical arm with four degrees of freedom, such as, for example:
- the possibility of rotating the radiation head 3' about the axis of rotation R (so-called roll axis);
- the possibility of rotating the radiation head 3' with respect to the first section of arm ("link") 23 about the first pitching axis AB1;
- the possibility of rotating the first 23 and the second 25 section of arm with respect to each other about the second pitching axis AB2;
- the possibility of rotating the first 23 and the second 25 section of arm and the radiation head 3' with respect to the base 50' about the substantially vertical axis AI.

According to embodiments not illustrated the movement system can comprise an anthropomorphic mechanical arm also with less than three or more than three degrees of freedom, that is to say, controlled axes.

According to embodiments not illustrated the substantially point-like, globular or rounded bodies 1100, or other real position markers, such as, for example, single rods, bars and lines, can be fixed directly to the probe 13, to the radiation head 3 or to the pointer 17 without the fork-like frame 112.

According to embodiments not illustrated each real position marker 110, 110', 110" can comprise five or more substantially point-like, globular or rounded bodies such as, for example, the above-mentioned balls 1100.

According to examples not illustrated the system 1 and the diagnostics process described above for obtaining a three-dimensional model, for example virtual or digital, of the inside of the body of the patient P can also be used for applications other than intraoperative radiotherapy.

The fact that expressions appear in various parts of the description does not imply that they are necessarily referred only to the same embodiment.

Moreover, when a characteristic, element or structure is described in relation to a particular embodiment, it should be noted that it falls within the skills the average technician to apply the characteristic, element or structure to other embodiments.

Numerical references which differ only in terms of different superscripts, e.g. 21′, 21ʺ, 21‴, indicate, unless specified otherwise, different variants of an element named in the same way.

For example, any materials and dimensions may be used, depending on the technical requirements.

It should be understood that an expression of the type "A *comprises* B, C, D" or "A *is formed by* B, C, D" also comprises and describes the particular case in which "A *consists of B,* C, D".

The expression *"A comprises an element B"* is to be understood as *"A comprises one or more elements B"* unless otherwise specified.

## Claims

1. A radiotherapy system (1) comprising:
- a radiation head (3);
- a movement system (7);
- a diagnostics subsystem for images (22) in turn comprising at least one probe (13);
- a position detection subsystem (11);
and wherein:
- the at least one probe (13) is designed for acquiring a plurality of images (IM_1, IM_2, IM_i, IM_N) of internal sections of a body to be treated (P);
- the position detection subsystem (11) is programmed for detecting the position in space of the probe (13) whilst it acquires each of said images (IM_1, IM_2, IM_i, IM_N);
- the movement system (7) is programmed for moving the radiation head (3) and performing a predetermined treatment on said body to be treated (P) on the basis of said images (IM_1, IM_2, IM_i, IM_N),
**characterized in that**
the at least one probe (13) is equipped with a pressure detector for detecting if the pressure with which an operator presses the probe (13) against the body to be treated (P) whilst it acquires one or more of said images (IM_1, IM_2 ... IM_i... IM_N) is equal to or greater than a predetermined pressure threshold, where said images (IM_1, IM_2 ... IM_i... IM_N) are preferably ultrasound.

2. The system (1) according to claim 1, comprising a treatment support (15) to which is fixed at least one real position marker of a first type (110) and at least one real position marker of a second type (110A), and wherein:
- a body to be treated (P) is fixed to the treatment support (15);
- the position detection subsystem (11) is programmed for measuring the position in space of the at least one real position marker of a first type (110) according to a first reference system of linear and/or angular coordinates (X, Y, Z; α, β, γ) of the position detection subsystem (11);
- the diagnostics subsystem for images (22) is designed for detecting the at least one real position marker of a second type (110A) and at least part of the body to be treated (P) and determining the position in space of the at least one real marker (110A) with respect to the at least part of the body to be treated (P) according to a second reference system of linear and/or angular coordinates (X", Y", Z"; α", β", γ") of the diagnostics subsystem for images (22);
- the system (1) is programmed for determining, on the basis of the measurements of the position detection subsystem (11) and the diagnostics subsystem for images (22), the position in space of the at least part of the body to be treated (P) in the first reference system of linear and/or angular coordinates (X, Y, Z; α, β, γ) of the position detection subsystem (11).

3. The system (1) according to claim 1 or 2, wherein the position detection subsystem (11) comprises one or more of the following subsystems for measurement of distance and dimensions: a stereoscopic optical system, a radar system with electromagnetic waves not in the visible light band, a radar system with electromagnetic and/or acoustic waves, a radar system with laser emission, a mechanical arm (19), a Cartesian and/or polar mechanical manipulator.

4. The system (1) according to any one of claims 1 to 3, wherein the position detection subsystem (11) comprises at least one real position marker of a first and/or second type (110, 110A) in turn comprising one or more of the following elements, designed for being detected, respectively, by the position detection subsystem (11) and/or by the diagnostics subsystem for images (22): at least one ball (1100,1100A), at least three balls (1100, 1100A), at least six balls (1100, 1100A), one of more globular or squat bodies, at least one rod, at least three rods, at least six rods, a body with a substantially polyhydric shape if necessary with at least three or at least six vertices, one or more emitters for electromagnetic signals not necessarily in the visible light band, one or more emitters of radio signals, one or more emitters of acoustic signals.

5. The system (1) according to any one of claims 1 to 4, comprising one or more pointers (17) each of which is designed to draw, mark or indicate zones of interest about a surgical incision made on the body to be treated (P), and wherein:
- the least one real position marker (110) is fixed to each pointer (17);
- each pointer (17) comprises one or more of the following elements: a pencil, pen or marker pen designed to make marks on the body of the patient, a luminous or laser stylus or marker (170) designed for projecting a luminous mark on the body of the patient.

6. A computer program comprising instructions which, when executed on a logic unit (21, 118) connected to said system (1) according to one or more of claims 1 to 5,cause the logic unit to carry out a process for performing radiotherapy treatment, comprising the following operations:
- by means of the at least one probe (13) acquiring a plurality of images (IM_1, IM_2, IM_i, IM_N) of internal sections of a body to be treated (P);
- by means of the position detection subsystem (11) detecting the position in space of the probe (13) whilst it acquires each of said images (IM_1, IM_2, IM_i, IM_N);
- on the basis of said images (IM_1, IM_2, IM_i, IM_N) and by means of the movement system (7) moving the radiation head (3) to a predetermined position in the space and performing a predetermined treatment on said body to be treated (P).

## Patentansprüche

1. Strahlentherapiesystem (1), umfassend:
- einen Strahlenkopf (3);
- ein Bewegungssystem (7);
- ein Diagnoseteilsystem für Bilder (22), das wiederum mindestens eine Sonde (13) umfasst;
- ein Positionserfassungsteilsystem (11);
und wobei:
- die mindestens eine Sonde (13) zum Erlangen einer Vielzahl von Bildern (IM_1, IM_2, IM_i, IM_N) von inneren Bereichen eines zu behandelnden Körpers (P) ausgelegt ist;
- das Positionserfassungsteilsystem (11) zum Erfassen der Position der Sonde (13) im Raum programmiert ist, während sie jedes der Bilder (IM_1, IM_2, IM_i, IM_N) erlangt;
- das Bewegungssystem (7) zum Bewegen des Strahlungskopfes (3) und Durchführen einer vorbestimmten Behandlung an dem zu behandelnden Körper (P) auf der Grundlage der Bilder (IM_1, IM_2, IM_i, IM_N) programmiert ist,
**dadurch gekennzeichnet, dass**
die mindestens eine Sonde (13) mit einem Druckerfasser ausgestattet ist, um zu erfassen, ob der Druck, mit dem ein Benutzer die Sonde (13) gegen den zu behandelnden Körper (P) drückt, während sie ein oder mehrere der Bilder (IM_1, IM_2 ... IM_i... IM_N) erlangt, gleich oder größer als eine vorbestimmte Druckschwelle ist, wobei die Bilder (IM_1, IM_2... IM_i... IM_N) vorzugsweise Ultraschall sind.

2. System (1) nach Anspruch 1, umfassend eine Behandlungsstütze (15), an der mindestens eine echte Positionsmarkierung eines ersten Typs (110) und mindestens eine echte Positionsmarkierung eines zweiten Typs (110A) angebracht sind, und wobei:
- ein zu behandelnder Körper (P) an der Behandlungsstütze (15) angebracht ist;
- das Positionserfassungsteilsystem (11) zum Messen der Postion der mindestens einen echten Positionsmarkierung eines ersten Typs (110) im Raum gemäß einem ersten Referenzsystem linearer und/oder winkliger Koordinaten (X, Y, Z; α, β, γ) des Positionserfassungsteilsystems (11) programmiert ist;
- das Diagnoseteilsystem für Bilder (22) zum Erfassen der mindestens einen echten Positionsmarkierung eines zweiten Typs (110A) und mindestens eines Teils des zu behandelnden Körpers (P) und Bestimmen der Position der mindestens einen echten Markierung (110A) im Raum in Bezug auf den mindestens einen zu behandelnden Körper (P) gemäß einem zweiten Referenzsystem linearer und/oder winkliger Koordinaten (X, Y, Z; α, β, γ) des Diagnoseteilsystems für Bilder (22) ausgelegt ist;
- das System (1) zum Bestimmen, auf der Grundlage der Messungen des Positionserfassungsteilsystems (11) und des Diagnoseteilsystems für Bilder (22), der Postion des mindestens einen zu behandelnden Körpers (P) im Raum im ersten Referenzsystem linearer und/oder winkliger Koordinaten (X, Y, Z; α, β, γ) des Positionserfassungsteilsystems (11) programmiert ist.

3. System (1) nach Anspruch 1 oder 2, wobei das Positionserfassungsteilsystem (11) ein oder mehrere der folgenden Teilsysteme zum Messen eines Abstandes und von Abmessungen umfasst: ein stereoskopisches optisches System, ein Radarsystem mit elektromagnetischen Wellen die nicht im sichtbaren Lichtband sind, ein Radarsystem mit elektromagnetischen und/oder akustischen Wellen, ein Radarsystem mit Laseremission, einen mechanischen Arm (19), einen kartesischen und/oder polaren mechanischen Manipulator.

4. System (1) nach einem der Ansprüche 1 bis 3, wobei das Positionserfassungsteilsystem (11) mindestens eine echte Positionsmarkierung eines ersten und/oder zweiten Typs (110, 110A) umfasst, die wiederum eines oder mehrere der folgenden Elemente umfasst, die ausgelegt sind, um jeweils von dem Positionserfassungsteilsystem (11) und/oder dem Diagnoseteilsystem für Bilder (22) erfasst zu werden: mindestens einen Ball (1100, 1100A), mindestens drei Bälle (1100, 1100A), mindestens sechs Bälle (1100, 1100A), einen oder mehrere kugelförmige oder gedrungene Körper, mindestens einen Stab, mindestens drei Stäbe, mindestens sechs Stäbe, einen Körper mit einer im Wesentlichen mehrwertigen Form, soweit erforderlich, mit mindestens drei oder mindestens sechs Scheitelpunkten, einen oder mehrere Sender für elektromagnetische Signale, die nicht unbedingt im sichtbaren Lichtband sind, einen oder mehrere Sender von Funksignalen, einen oder mehrere Sender von akustischen Signalen.

5. System (1) nach einem der Ansprüche 1 bis 4, umfassend einen oder mehrere Zeiger (17), von denen jeder dazu ausgelegt ist, interessierende Zonen bezüglich eines an dem zu behandelnden Körper (P) ausgeführten chirurgischen Schnitts zu zeichnen, zu markieren oder anzugeben, und wobei:
- die mindestens eine echte Positionsmarkierung (110) an jedem Zeiger (17) angebracht ist;
- jeder Zeiger (17) eines oder mehrere der folgenden Elemente umfasst: einen Bleistift, Stift oder Markierstift, der dazu ausgelegt ist, Markierungen an dem Körper des Patienten zu machen, einen Leucht- oder Laserschreibstift oder Markierer (170), der dazu ausgelegt ist, eine Leuchtmarkierung auf den Körper des Patienten zu projizieren.

6. Computerprogramm, das Anweisungen umfasst, die, wenn sie auf einer mit dem System (1) nach einem der Ansprüche 1 bis 5 verbundenen Logikeinheit (21, 118) ausgeführt werden, die Logikeinheit veranlassen, einen Prozess zum Durchführen einer Strahlentherapiebehandlung vorzunehmen, der die folgenden Vorgänge umfasst:
- mittels der mindestens einen Sonde (13), Erlangen einer Vielzahl von Bildern (IM_1, IM_2, IM_i, IM_N) von inneren Bereichen eines zu behandelnden Körpers (P);
- mittels des Positionserfassungsteilsystems (11), Erfassen der Position der Sonde (13) im Raum, während sie jedes der Bilder (IM_1, IM_2, IM_i, IM_N) erlangt;
- auf der Grundlage der Bilder (IM_1, IM_2, IM_i, IM_N) und mittels des Bewegungssystems (7), Bewegen des Strahlungskopfes (3) zu einer vorbestimmten Position im Raum und Durchführen einer vorbestimmten Behandlung an dem zu behandelnden Körper (P).

## Revendications

1. Système de radiothérapie (1) comprenant :
- une tête de rayonnement (3) ;
- un système de déplacement (7) ;
- un sous-système de diagnostic pour des images (22) comprenant à son tour au moins une sonde (13) ;
- un sous-système de détection de position (11) ;
et dans lequel :
- la au moins une sonde (13) est conçue pour obtenir une pluralité d'images (IM_1, IM_2, IM_i, IM_N) de sections internes d'un corps à traiter (P) ;
- le sous-système de détection de position (11) est programmé pour détecter la position dans l'espace de la sonde (13) tandis qu'elle obtient chacune desdites images (IM_1, IM_2, IM_i, IM_N) ;
- le système de déplacement (7) est programmé pour déplacer la tête de rayonnement (3) et effectuer un traitement prédéterminé sur ledit corps à traiter (P) sur la base desdites images (IM_1, IM_2, IM_i, IM_N),
**caractérisé en ce que**
la au moins une sonde (13) est équipée d'un détecteur de pression pour détecter si la pression avec laquelle un opérateur appuie sur la sonde (13) contre le corps à traiter (P) tandis qu'elle obtient une ou plusieurs desdites images (IM_1, IM_2, ... IM_i, ... IM_N) est égale ou supérieure à un seuil de pression prédéterminé, où lesdites images (IM_1, IM_2, ... IM_i, ... IM_N) sont de préférence à ultrasons.

2. Système (1) selon la revendication 1, comprenant un support de traitement (15) auquel est fixé au moins un marqueur de position réelle d'un premier type (110) et au moins un marqueur de position réelle d'un second type (110A), et dans lequel :
- un corps à traiter (P) est fixé au support de traitement (15) ;
- le sous-système de détection de position (11) est programmé pour mesurer la position dans l'espace du au moins un marqueur de position réelle d'un premier type (110) selon un premier système de référence de coordonnées linéaires et/ou angulaires (X, Y, Z ; α, β, γ) du sous-système de détection de position (11) ;
- le sous-système de diagnostic pour des images (22) est conçu pour détecter le au moins un marqueur de position réelle d'un second type (110A) et au moins une partie du corps à traiter (P) et déterminer la position dans l'espace du au moins un marqueur de position réelle (110A) par rapport à la au moins une partie du corps à traiter (P) selon un second système de référence de coordonnées linéaires et/ou angulaires (X, Y, Z ; α, β, γ) du sous-système de diagnostic pour des images (22) ;
- le système (1) est programmé pour déterminer, sur la base des mesures du sous-système de détection de position (11) et du sous-système de diagnostic pour des images (22), la position dans l'espace de la au moins une partie du corps à traiter (P) dans le premier système de référence de coordonnées linéaires et/ou angulaires (X, Y, Z ; α, β, γ) du sous-système de détection de position (11).

3. Système (1) selon la revendication 1 ou 2, dans lequel le sous-système de détection de position (11) comprend un ou plusieurs des sous-systèmes suivants pour la mesure de distance et de dimensions : un système optique stéréoscopique, un système de radar avec des ondes électromagnétiques pas dans la bande de lumière visible, un système de radar avec des ondes électromagnétiques et/ou acoustiques, un système de radar avec une émission laser, un bras mécanique (19), un manipulateur mécanique cartésien et/ou polaire.

4. Système (1) selon l'une quelconque des revendications 1 à 3, dans lequel le sous-système de détection de position (11) comprend au moins un marqueur de position réelle d'un premier et/ou second type (110, 110A) comprenant à son tour un ou plusieurs des éléments suivants, conçus pour être détectés, respectivement, par le sous-système de détection de position (11) et/ou par le sous-système de diagnostic pour des images (22) : au moins une bille (1100, 1100A), au moins trois billes (1100, 1100A), au moins six billes (1100, 1100A), un ou plusieurs corps globulaires ou trapus, au moins une tige, au moins trois tiges, au moins six tiges, un corps avec une forme sensiblement polyhydrique le cas échéant avec au moins trois ou au moins six sommets, un ou plusieurs émetteurs pour des signaux électromagnétiques pas nécessairement dans la bande de lumière visible, un ou plusieurs émetteurs de signaux radio, un ou plusieurs émetteurs de signaux acoustiques.

5. Système (1) selon l'une quelconque des revendications 1 à 4, comprenant un ou plusieurs pointeurs (17) dont chacun est conçu pour tracer, marquer ou indiquer des zones d'intérêt autour d'une incision chirurgicale faite sur le corps à traiter (P), et dans lequel :
- le au moins un marqueur de position réelle (110) est fixé à chaque pointeur (17) ;
- chaque pointeur (17) comprend un ou plusieurs des éléments suivants : un crayon, stylo ou marqueur conçu pour faire des marques sur le corps du patient, un stylet ou marqueur lumineux ou laser (170) conçu pour projeter une marque lumineuse sur le corps du patient.

6. Programme informatique comprenant des instructions qui, lorsqu'elles sont exécutées sur une unité logique (21, 118) raccordée audit système (1) selon l'une ou plusieurs des revendications 1 à 5, amènent l'unité logique à exécuter un processus pour effectuer un traitement de radiothérapie, comprenant les opérations suivantes consistant à :
- au moyen de la au moins une sonde (13), obtenir une pluralité d'images (IM_1, IM_2, IM_i, IM_N) de sections internes d'un corps à traiter (P) ;
- au moyen du sous-système de détection de position (11), détecter la position dans l'espace de la sonde (13) tandis qu'elle obtient chacune desdites images (IM_1, IM_2, IM_i, IM_N) ;
- sur la base desdites images (IM_1, IM_2, IM_i, IM_N) et au moyen du système de déplacement (7), déplacer la tête de rayonnement (3) vers une position prédéterminée dans l'espace et effectuer un traitement prédéterminé sur ledit corps à traiter (P).
